# EUROPEAN PATENT APPLICATION

(11) **EP 4 356 855 A1**
(43) Date of publication of application: **24.04.2024**
(21) Application number: 21951992.3
(22) Date of filing: 02.08.2021
(51) Int. Cl.: A61B 18/14, A61N 1/36, A61N 1/05, A61B 18/00

(54) **ELECTRODE DEVICE FOR BLOCKING OR CONTROLLING NERVES IN BODY**

(30) Priority: 29.07.2021 KR 20210099569
(71) Applicant: Deepqure Inc., Seoul 03136 (KR)
(72) Inventor: BACH, Du Jin, Seongnam-si Gyeonggi-do 13506 (KR); JO, Seok Hyeon, Namyangju-si Gyeonggi-do 12167 (KR)
(74) Representative: Manitz Finsterwald Patent- und Rechtsanwaltspartnerschaft mbB
(86) International application number: PCT/KR2021/010050
(87) International publication number: WO 2023/008622

(57) **Abstract**

An electrode device for nerve denervation or modulation in vivo includes a main body including a shaft; an electrode unit formed to be drawn out from one end of the shaft and configured to denervate or modulate at least some of nerves on a tube in the body; an electrode guide coupled to the end of the electrode unit and configured to guide the electrode unit to be brought into contact with the tube in the body; an electrode guide driving unit configured to move the electrode guide in forward and backward directions; and an electrode driving unit configured to move the electrode guide in the forward and backward directions in conjunction with the electrode guide driving unit.

## Description

### TECHNICAL FIELD

The present disclosure relates to an electrode device for nerve denervation or modulation in vivo.

### BACKGROUND

A denervation is a surgical procedure intended to control an abnormally overactive autonomic nervous system by damaging specific nerves. For example, a renal denervation can treat hypertension and heart diseases by damaging renal sympathetic nerves directed to the kidney, and a pulmonary denervation can treat lung diseases by damaging parasympathetic nerves directed to the lung.

Nerves usually enclose the outer walls of tubes, such as blood vessels, bronchial tubes, etc., and it may be necessary to enclose the outer walls of tubes to measure signals from the nerves or transmit electrical impulses or various energies to the nerves to damage or destroy the nerves. For example, when a surgical procedure is performed on the renal artery, the main renal artery which is a procedure target has a diameter of from 5 mm to 7 mm, and the accessory renal artery having a diameter of from 1 mm to 2 mm may also be a procedure target. Also, the artery with distributed nerves varies in size from person to person and has different sizes depending on the location.

When the surgical procedure is performed as described above, it is important to delicately locate a component including an electrode to be formed at the end of a catheter so as to enclose the outer wall of the artery. Specifically, in order to effectively denervate or modulate the nerves, the component needs to enclose the outer wall of the artery with distributed nerves in a circumferential direction. Also, it is necessary to reliably and rapidly enclose the artery with the component including the electrode. In particular, it is important to safely and accurately adhere the electrode-formed component to the outer wall of the tube in the body so as not to damage the tube in the body, which can be easily damaged by external stimuli.

### DISCLOSURE OF THE INVENTION

### PROBLEMS TO BE SOLVED BY THE INVENTION

The present disclosure is conceived to provide an electrode device having a component that guides a plurality of unit elements to enclose the circumference of a tube in the body.

Also, the present disclosure is conceived to provide an electrode device configured to accurately bring a component including an electrode into close contact with an outer wall of the tube in the body without damaging the tube which can be easily damaged by external stimuli.

The problems to be solved by the present disclosure are not limited to the above-described problems. There may be other problems to be solved by the present disclosure.

### MEANS FOR SOLVING THE PROBLEMS

According to an aspect of the present disclosure, an electrode device for nerve denervation or modulation in vivo includes a main body including a shaft; an electrode unit formed to be drawn out from one end of the shaft and configured to denervate or modulate at least some of nerves on a tube in the body; an electrode guide coupled to the end of the electrode unit and configured to guide the electrode unit to be brought into contact with the tube in the body; an electrode guide driving unit configured to move the electrode guide in forward and backward directions; and an electrode driving unit configured to move the electrode guide in the forward and backward directions in conjunction with the electrode guide driving unit. The electrode driving unit includes a tensile force maintaining unit connected to one end of the electrode unit and configured to provide a tensile force to the electrode unit; and a moving unit that moves the tensile force maintaining unit in the forward direction until the electrode guide encloses the circumference of the tube in the body in a state where the moving unit is connected to the tensile force maintaining unit, and then is disconnected from the tensile force maintaining unit. After the tensile force maintaining unit and the moving unit are disconnected from each other, an electrode connection portion connected to one end of the electrode unit moves in the backward direction.

The above-described aspects are provided by way of illustration only and should not be construed as liming the present disclosure. Besides the above-described embodiments, there may be additional embodiments described in the accompanying drawings and the detailed description.

### EFFECTS OF THE INVENTION

According to any one of the above-described aspects of the present disclosure, an electrode guide is located close to a tube in the body and then gradually brings an electrode unit into close contact with an outer wall of the tube, and, thus, an electrode driving unit after an electrode guide can safely and accurately bring a component including an electrode into close contact with the outer wall of the tube without damaging the tube which can be easily damaged by external stimuli.

### BRIEF DESCRIPTION OF THE DRAWINGS

**FIG. 1** is a side view of an electrode device according to an embodiment of the present disclosure.
**FIG. 2** illustrates a state where an electrode guide illustrated in **FIG. 1** guides and locates an electrode unit to enclose a blood vessel according to an embodiment of the present disclosure.
**FIG. 3A** illustrates an operation process of the electrode guide according to an embodiment of the present disclosure.
**FIG. 3B** illustrates an operation process of the electrode guide according to an embodiment of the present disclosure.
**FIG. 3C** illustrates an operation process of the electrode guide according to an embodiment of the present disclosure.
**FIG. 3D** illustrates an operation process of the electrode guide according to an embodiment of the present disclosure.
**FIG. 3E** illustrates an operation process of the electrode guide according to an embodiment of the present disclosure.
**FIG. 4** is an exploded perspective view illustrating a portion of joint units illustrated in **FIG. 2****.**
**FIG. 5** is a cross-sectional view of an electrode guide driving unit located inside a main body illustrated in **FIG. 1****.**
**FIG. 6A** illustrates an operation process of an electrode driving unit according to an embodiment of the present disclosure.
**FIG. 6B** illustrates an operation process of the electrode driving unit according to an embodiment of the present disclosure.
**FIG. 6C** illustrates an operation process of the electrode driving unit according to an embodiment of the present disclosure.
**FIG. 6D** illustrates an operation process of the electrode driving unit according to an embodiment of the present disclosure.
**FIG. 6E** illustrates an operation process of the electrode driving unit according to an embodiment of the present disclosure.
**FIG. 6F** illustrates an operation process of the electrode driving unit according to an embodiment of the present disclosure.
**FIG. 7** is an example diagram provided to explain the electrode driving unit according to another embodiment of the present disclosure.

### BEST MODE FOR CARRYING OUT THE INVENTION

Hereafter, example embodiments will be described in detail with reference to the accompanying drawings so that the present disclosure may be readily implemented by those skilled in the art. However, it is to be noted that the present disclosure is not limited to the example embodiments but can be embodied in various other ways. In the drawings, parts irrelevant to the description are omitted for the simplicity of explanation, and like reference numerals denote like parts through the whole document.

Through the whole document, the term "connected to" or "coupled to" that is used to designate a connection or coupling of one element to another element includes both a case that an element is "directly connected or coupled to" another element and a case that an element is "electronically connected or coupled to" another element via still another element. Further, it is to be understood that the term "comprises or includes" and/or "comprising or including" used in the document means that one or more other components, steps, operation and/or existence or addition of elements are not excluded in addition to the described components, steps, operation and/or elements unless context dictates otherwise and is not intended to preclude the possibility that one or more other features, numbers, steps, operations, components, parts, or combinations thereof may exist or may be added.

Hereinafter, an exemplary embodiment of the present disclosure will be described in detail with reference to the accompanying configuration views or process flowcharts.

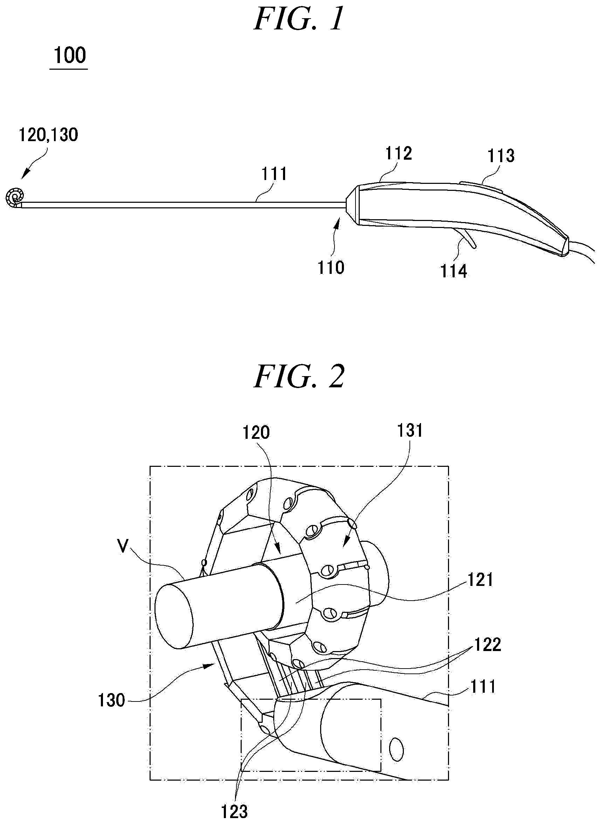

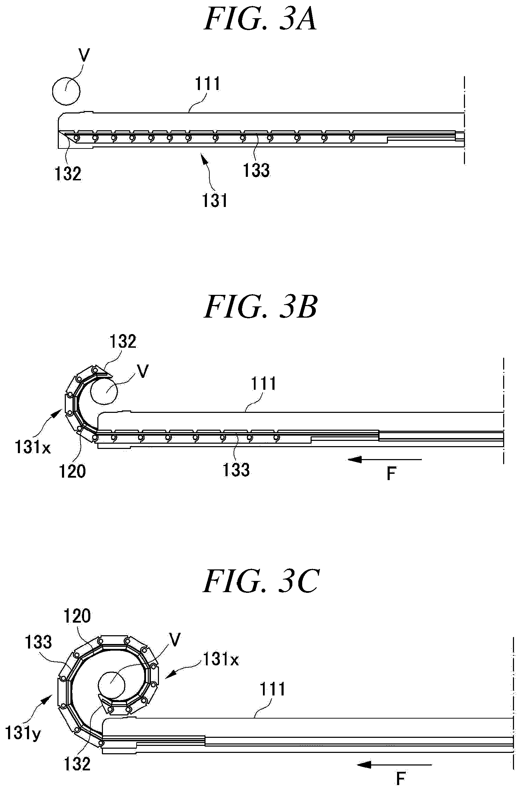

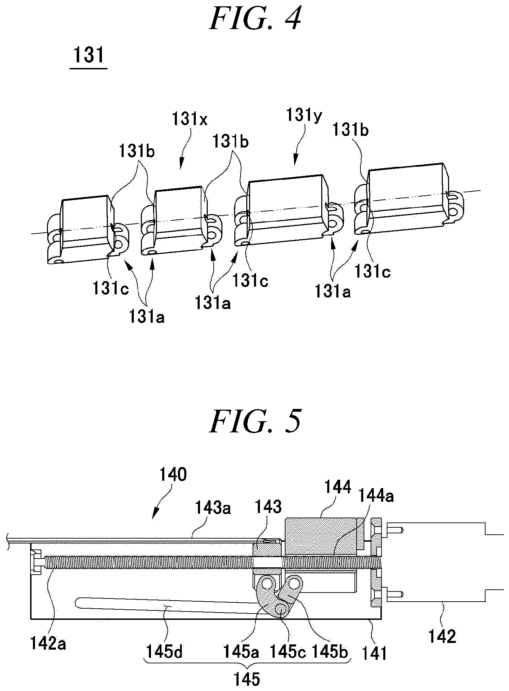

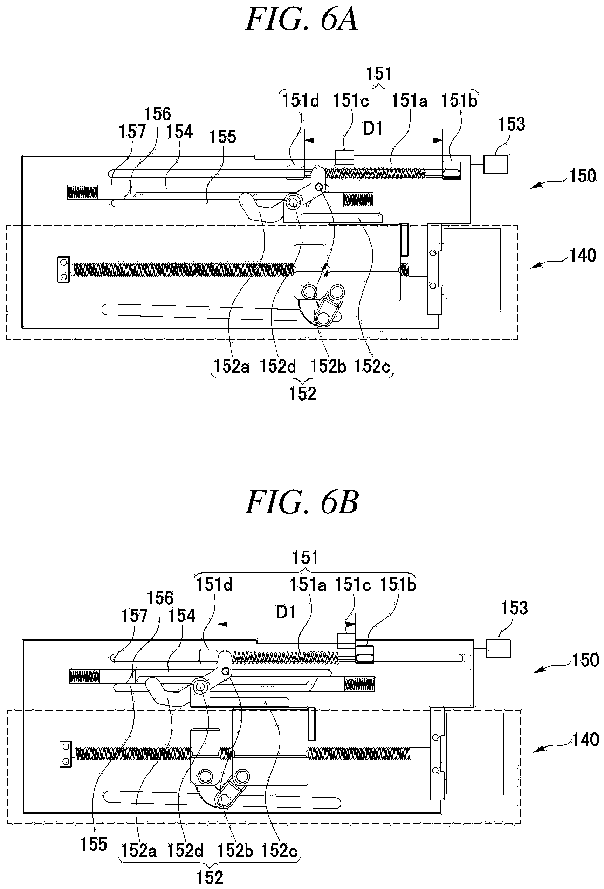

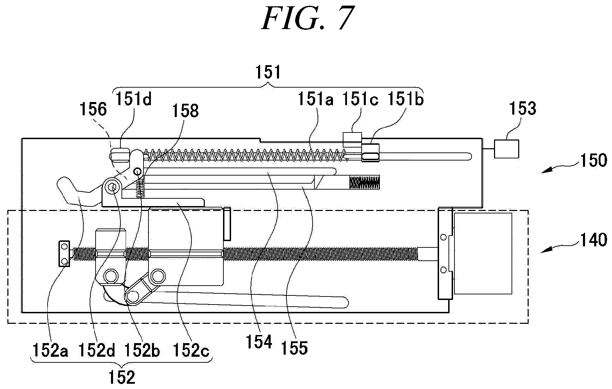

**FIG. 1** is a side view of an electrode device according to an embodiment of the present disclosure. **FIG. 2** illustrates a state where an electrode guide illustrated in **FIG. 1** guides and locates an electrode unit to enclose a blood vessel according to an embodiment of the present disclosure. **FIG. 3A** through **FIG. 3E** illustrates an operation process of the electrode guide according to an embodiment of the present disclosure. **FIG. 4** is an exploded perspective view illustrating a portion of joint units illustrated in **FIG. 2****.** **FIG. 5** is a cross-sectional view of an electrode guide driving unit located inside a main body illustrated in **FIG. 1****.** **FIG. 6A** through **FIG. 6F** illustrates an operation process of an electrode driving unit according to an embodiment of the present disclosure. **FIG. 7** is an example diagram provided to explain the electrode driving unit according to another embodiment of the present disclosure.

Referring to **FIG. 1**, the electrode device 100 includes the main body 110, the electrode unit 120 and the electrode guide 130, the electrode guide driving unit 140 and the electrode driving unit 150 disposed inside the main body 110.

The main body 110 may include a shaft 111 extending in one direction, a grip portion 112 connected to the shaft 111 so as to be gripped by an operator, a guide manipulation unit 113 formed on the grip portion 112 so as to manipulate an operation of the electrode guide 130, and an electrode manipulation unit 114 formed on the grip portion 112 so as to manipulate energy transfer to the electrode unit 120.

The components for driving and controlling the electrode unit 120 and the electrode guide 130 may be located inside the main body 110. For example, the electrode guide driving unit 140 configured to drive and control the electrode guide 130 and the electrode driving unit 150 configured to drive and control the electrode unit 120 may be disposed inside the main body 110.

The electrode unit 120 is formed to be drawn out from one end of the shaft 111 and configured to denervate or modulate at least part of nerves distributed on a tissue in the body including a tube depending on manipulation by the operator. The electrode unit 120 is accommodated inside the shaft 111 and when the electrode device 100 operates, the electrode unit 120 can be drawn out by means of the electrode guide 130 which will be described later.

Referring to **FIG. 2**, the electrode unit 120 may include a base unit 121, an electrode unit 122 and a sensor unit 123. In the electrode device 100, an electrode encloses an outer surface of a tube or tube-shaped tissue V in the body and energy can be transferred through the electrode. To this end, the base unit 121 may be formed as a flexible printed circuit board (PCB).

The electrode unit 122 may be composed of two electrodes extending parallel to each other on the base unit 121 on the base unit 121. In the present embodiment, the base unit 121 and the electrode unit 122 may be configured to extend in a circumferential direction and enclose the tube in the body or the like.

The electrode unit 122 may be made of a material such as stainless steel or gold, which is harmless to the human body and conducts electricity well, in order to block or denervate or control or modulate the nerves.

Also, the electrode unit 122 may transfer various types of energy from an energy source generator. For example, the energy may include radio-frequency (RF) energy, electrical energy, laser energy, ultrasonic energy, high-intensity focused ultrasound energy, cryogenic energy and other heat energy.

Also, the electrode unit 122 may be implemented as a flexible PCB for transferring RF energy, a transducer for transferring ultrasonic energy or a metal electrode for transferring high-voltage energy and thus may transfer energy to damage the nerves.

Further, the sensor unit 123 may be formed on the base unit 121. For example, the sensor unit 123 may be a thermocouple that measures a temperature by contacting with the tube in the body or the like, and when neurotomy is performed with the electrode device 100, the sensor unit 123 may monitor a temperature of a treatment site. As another example, the sensor unit 123 may measure signals from the nerves on the tube.

The sensor unit 123 may be, for example, a thermocouple composed of a pair of copper and constantan.

The electrode guide 130 functions to bring the electrode unit 120 into contact with the tube in the body. The electrode guide 130 is coupled to the electrode unit 120 and deformed into a wound state to bring the electrode unit 120 into contact with the tube in the body.

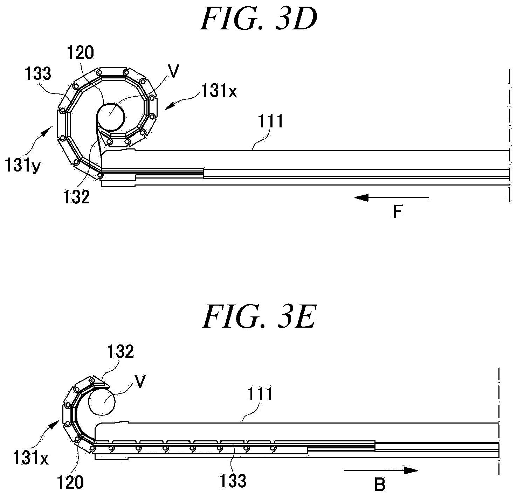

Referring to **FIG. 2** through **FIG. 4**, the electrode guide 130 includes a plurality of joint units 131. The plurality of joint units 131 forms a curved winding path P to enclose the circumference of the tube V in the body with the electrode unit 120 interposed therebetween. The state illustrated in **FIG. 2****,** **FIG. 3C** and **FIG. 3D** may be a state where the plurality of joint units 131 is disposed along the curved winding path P.

Referring to **FIG. 3A** through **FIG. 3E**, the electrode guide 130 may further include a tip joint 132 and a wire 133. The tip joint 132 may support the electrode unit 120 and may be coupled to the end of the plurality of joint units 131 connected sequentially to each other.

The tip joint 132 may be drawn out from one end of the shaft 111 earlier than the plurality of joint units 131. As illustrated in **FIG. 3D**, the tip joint 132 may be located close to the tube V in the body and may have a tapered shape that gradually decreases in width or thickness toward the end in order to suppress interference with the electrode unit 120 or maximize the surface enclosing the tube in the body. The end of the electrode unit 120 may be fastened and fixed to the tip joint 132.

The wire 133 may be formed to sequentially penetrate the plurality of joint units 131. Referring to **FIG. 4**, each joint unit 131 may have a through-hole 131c in a longitudinal direction to allow penetration of the wire 133.

The end of the wire 133 sequentially penetrating the through-holes 131c may be coupled and fixed to the tip joint 132, and the wire 133 can slide with respect to each joint unit 131 in the through-hole 131c in the longitudinal direction.

Therefore, the wire 133 can guide the plurality of joint units 131 and the tip joint 132 to be located on the winding path and provide a force of pulling the plurality of joint units 131 and the tip joint 132 in a direction to be wound around the tube V.

The wire 133 may be operated to protrude from one end of the shaft 111 together with to the plurality of joint units 131. Here, the wire 133 may be designed to protrude less than the plurality of joint units 131 per unit time and thus can provide a force of pulling the plurality of joint units 131 along a curved path.

Each join unit 131 may include hinge units 131a and winding support units 131b. The hinge units 131a are configured for rotatable connection to adjacent joints and may be formed on one or both sides of the joint unit 131 in the longitudinal direction in which the joint units 131 are connected parallel to each other.

As illustrated in **FIG. 4**, the hinge unit 131a may have a rotation axis in a direction intersecting the longitudinal direction so as to be connected to the hinge unit 131a of the adjacent joint unit 131. A hinge pin (not illustrated) may be inserted into and fastened to each hinge unit 131a in the direction of the rotation axis.

The winding support units 131b are configured to support the plurality of joint units 131 on the winding path and may be formed on one or both sides of the joint unit 131 in the longitudinal direction to support the adjacent joint unit 131.

As illustrated in **FIG. 2** and **FIG. 4**, the winding support unit 131b may be located adjacent to the hinge unit 131a in an inward direction of the electrode guide 130 (in a direction of winding the joint unit 131).

For example, the winding support unit 131b may be formed as a surface having a predetermined angle and area and supported by the adjacent winding support unit 131b in surface contact with each other, and, thus, a wound shape of the electrode guide 130 can be maintained.

The winding support unit 131b and the wire hole 131c may be formed at locations spaced apart from a rotation center of the hinge unit 131a in an inward direction toward the tube V in the body.

When the wire 133 is pulled backwards relative to the electrode guide 130 (when a length of the wire 133 drawn out from the shaft 111 is smaller than that of the joint units 131), a tensile force may be applied to the wire 133 in a direction of winding the electrode guide 130. On the other hand, the winding support units 131b may provide a force of supporting the joint units 131 to each other in a direction of suppressing winding of the electrode guide 130. Since the wire 133 and the winding support units 131b form a balanced force in opposite directions, the electrode guide 130 can be fixed on the winding path.

Further, the electrode guide 130 may include a first joint group 131x and a second joint group 131y. That is, the plurality of joint units 131 may be divided into the first joint group 131x and the second joint group 131y having different lengths.

Due to a difference in length, the first joint group 131x may form a first radius of curvature and the second joint group 131y may form a second radius of curvature greater than the first radius of curvature. As can be seen from **FIG. 3D**, the joint units having a relatively small length (the first joint group 131x) may form a smaller radius of curvature and the joint units having a relatively great length (the second joint group 131y) may form a greater radius of curvature.

When the joint units 131 located close to the tip joint 132 form a path having a smaller radius of curvature, a path along which the tip joint 132 enters a space between the tube in the body and the shaft 111 may be formed as shown in **FIG. 3D****.** Also, the electrode guide 130 including the joint units 131 may have an overall spiral shape.

Referring to **FIG. 3A** through **FIG. 3E**, the electrode guide 130 is accommodated together with the electrode unit 120 inside the shaft 111 and may protrude from one end in a forward direction F while being deformed into the wound state at the time of surgical procedure.

For example, when the plurality of joint units 131 is sequentially drawn out, the plurality of joint units 131 may move along the curved winding path due to a difference in displacement from the wire 133 and thus may overall enclose the tube V.

Further, the electrode guide 130 is spaced apart from an outer circumferential surface of the tube and the electrode unit 120 located inside the wound electrode guide 130 may be in close contact with the outer circumferential surface of the tube V.

The plurality of joint units 131 may be drawn out from the shaft 111 by means of the electrode guide driving unit 140 and wound in a direction to enclose the tube V. Accordingly, a space where the electrode guide 130 operates can be minimized, and an operation of denervating or modulating nerves can be performed safely and accurately in a narrow space.

Referring to **FIG. 5**, the electrode guide driving unit 140 may be configured to move the electrode guide 130 in forward and backward directions, and may include a frame 141, a motor unit 142, a rod block 143, a wire block 144 and a variable connection unit 145.

The frame 141 may be provided to be fixed inside the main body and may include a guide slot or guide shaft extending in the forward and backward directions.

The motor unit 142 may be connected to the frame 141 and may rotate a rotation shaft 142a rotatably supported by the frame 141. For example, the motor unit 142 may receive electrical energy to rotate the rotation shaft 142a.

One end of the rod block 143 may be connected to the joint unit 131. The rod block 143 may be moved in the forward and backward directions by means of the motor unit 142. Specifically, the rod block 143 may be moved in the forward and backward directions in engagement with the rotation shaft 142a extending in the forward and backward directions and having a thread thereon.

The rod block 143 may include a rod 143a, which is located inside the shaft 111 and extends in one direction (forward and backward directions) and supports the joint units 131, and a corrugated component slidably coupled to the guide slot or guide shaft of the frame 141.

In addition to the above-described rotation shaft 142a and motor unit 142, the electrode guide driving unit 140 according to the present disclosure may be configured to move the rod block 143 in the forward and backward directions by various linear actuation mechanisms. For example, the electrode guide driving unit 140 may include a linear actuator of cylinder type including a pneumatic, hydraulic or electric linear actuator, or a piezoelectric or ultrasonic linear actuator.

The wire block 144 may be formed to support the wire 133 and moved in the forward and backward directions in conjunction with the rod block 143. The wire block 144 may include a corrugated component slidably inserted into the guide slot or guide shaft and a sliding hole 144a slidably accommodating the rotation shaft 142a, and may move in the forward and backward directions in parallel to the rod block 143

The variable connection unit 145 may connect the rod block 143 and the wire block 144 to each other and vary a distance between the rod block 143 and the wire block 144. To this end, the variable connection unit 145 may include a rod link 145a, a wire link 145b, a hinge pin 145c and a pin slot 145d.

The rod link 145a and the wire link 145b may be rotatably connected to the rod block 143 and the wire block 144, respectively. Also, the rod link 145a and the wire link 145b may be rotatably connected to each other by the hinge pin 145c.

The pin slot 145d is formed to slidably accommodate the hinge pin 145c. Specifically, the pin slot 145d is formed to extend at a predetermined tilt angle with respect to the forward and backward directions. The pin slot 145d may be formed in the frame 141.

Meanwhile, the electrode unit 120 may be drawn out from the shaft 111 by means of the electrode driving unit 150 and may be wound in the direction to enclose the tube V by means of the electrode guide 130. Specifically, when the electrode unit 120 moves together with the electrode guide 130 in the forward direction along the curved winding path and completely drawn out from the shaft 111 and disposed, the electrode unit 120 may be gradually brought into close contact with the tube V in the body under the control of the electrode driving unit 150. Therefore, in a state where the electrode unit 120 is stably in contact with the tube V in the body without damaging the tube V in the body, an operation of denervating or modulating nerves can be performed.

Referring to **FIG. 6A**, the electrode driving unit 150 may be configured to move the electrode unit 120 in the forward and backward directions in conjunction with the electrode guide driving unit 140. The electrode driving unit 150 may include a tensile force maintaining unit 151, a moving unit 152, a reducer 153, a forward movement rail 154, a backward movement rail 155, a connection rail 156 connecting the forward movement rail 154 and the backward movement rail 155, and a second stopper 157. Herein, the forward movement rail 154 and the backward movement rail 155 may have the same length.

The tensile force maintaining unit 151 may be connected to one end of the electrode unit 120 and may provide a tensile force to the electrode unit 120. The tensile force maintaining unit 151 may include a first spring 151a, the protrusion 151b upwardly protruding from one side, a first stopper 151c an electrode connection portion 151d on the other side.

The first spring 151a may provide a tensile force to the electrode unit 120, and the first stopper 151c may block movement of the protrusion 151b when the tensile force maintaining unit 151 moves in the forward direction to generate the tensile force of the first spring 151a.

The electrode connection portion 151d may be connected to one side of the electrode unit 120 and transfer the tensile force of the first spring 151a to the electrode unit 120. The electrode connection portion 151d may move in the backward direction due to a decrease in tensile force of the first spring 151a when the tensile force maintaining unit 151 and the moving unit 152 are disconnected from each other.

The moving unit 152 may move the tensile force maintaining unit 151 in the forward direction until the electrode guide 130 encloses the circumference of the tube V in the body in a state where the moving unit 152 is connected to the tensile force maintaining unit 151, and then may be disconnected from the tensile force maintaining unit 151.

The moving unit 152 may include a connection portion 152a for connection to the tensile force maintaining unit 151, the pin 152b, a support 152c and a hinge 152d.

The pin 152b may be formed on one side of the connection portion 152a, and may move in the forward direction along the forward movement rail 154 or may move in backward direction along the backward movement rail 155. Accordingly, the moving unit 152 may move in the forward direction together with the tensile force maintaining unit 151 along the forward movement rail 154 through the pin 152b, and may move in the backward direction along the backward movement rail 155 after being disconnected from the tensile force maintaining unit 151.

The support 152c may be connected to the electrode guide driving unit 140. For example, the support 152c may be connected to the wire block 144.

The hinge 152d is configured to make the connection portion 152a rotate, and when the pin 152b moves from the forward movement rail 154 to the connection rail 156, the hinge 152d rotates and the connection portion 152a may be disconnected from the tensile force maintaining unit 151. Therefore, after the connection portion 152a is disconnected from the tensile force maintaining unit 151, each of the electrode unit 120 and the electrode guide 130 may move.

The reducer 153 may reduce the tensile force of the first spring 151a when the tensile force maintaining unit 151 and the moving unit 152 are disconnected from each other.

Specifically, when the tensile force maintaining unit 151 and the moving unit 152 are disconnected from each other, the tensile force of the first spring 151a is transferred all at once to the electrode unit 120, and, thus, the electrode unit 120 is brought into close contact with the tube V, which may cause damage to the tube V. Therefore, in the present disclosure, the reducer 153 gradually reduces the tensile force of the first spring 151a so that damage to the tube V can be suppressed.

When the pin 152b moves in the backward direction, the second stopper 157 may suppress the pin 152b not to move again along the connection rail 156. The second stopper 157 may block the connection rail 156 when the pin 152b is located on the backward movement rail 155 through the connection rail 156.

Hereafter, driving of the electrode unit 120 by means of the electrode driving unit 150 will be described with reference to **FIG. 6A** through **FIG. 6D****.** **FIG. 6A** through **FIG. 6F** illustrate states corresponding to the states illustrated in **FIG. 3A** through **FIG. 3E****.**

The electrode driving unit 150 and the electrode guide driving unit 140 illustrated in **FIG. 6A** may be in a state right before forward movement starts or right after backward movement ends. Therefore, as illustrated in **FIG. 3A**, the electrode unit 120 and the electrode guide 130 may be in a state right before enclosing the circumference of the tube V in the body or right after the electrode unit 120 and the electrode guide 130 having enclosed the circumference of the tube V in the body are transitioned to the state before enclosing the tube V. That is, the electrode unit 120 and the electrode guide 130 may be in a state right before or right after neurotomy is performed with the electrode device 100.

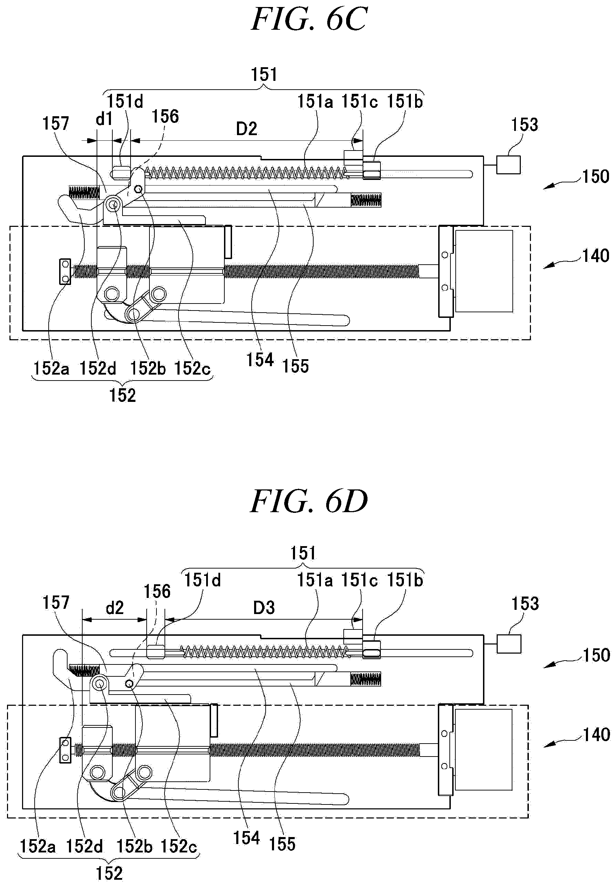

Referring to **FIG. 6B** and **FIG. 6C**, the electrode driving unit 150 may move in the forward direction along a path provided by the forward movement rail 154 together with the electrode guide driving unit 140 moving in the forward direction. As illustrated in **FIG. 3B** and **FIG. 3C**, due to forward movement of the electrode driving unit 150 and the electrode guide driving unit 140, the electrode unit 120 and the electrode guide 130 may be drawn out from the shaft 111 in the forward direction F and wound to enclose the circumference of the tube V in the body.

Specifically, when the electrode guide driving unit 140 is moved in the forward direction by driving of the motor unit 142, the tensile force maintaining unit 151 is also moved in the forward direction through the moving unit 152.

That is, as the electrode guide driving unit 140 moves in the forward direction, the pin 152b of the moving unit 152 connected to the electrode guide driving unit 140 may move in the forward direction along the forward movement rail 154. Here, the electrode guide 130 is drawn out from the shaft 111 in the forward direction F and the tensile force maintaining unit 151 connected to the moving unit 152 moves in the forward direction, and, thus, the electrode unit 120 of which one end is connected to the electrode connection portion 151d may also be drawn out from the shaft 111.

Here, the first stopper 151c of the tensile force maintaining unit 151 may block movement of the protrusion 151b when the tensile force maintaining unit 151 moves in the forward direction. Referring to **FIG. 6B**, forward movement of the protrusion 151b on one side of the tensile force maintaining unit 151, which moves together with the electrode guide driving unit 140, is blocked by the first stopper 151c, but the electrode connection portion 151d on the other side of the tensile force maintaining unit 151 can move in the forward direction together with the moving unit 152.

As illustrated in **FIG. 6C**, forward movement of the protrusion 151b of the tensile force maintaining unit 151 is blocked by the first stopper 151c, but the electrode connection portion 151d of the tensile force maintaining unit 151 moves in the forward direction together with the moving unit 152, and, thus, a length of the first spring 151a may increase (D1->D2) and a tensile force may be generated.

Referring to **FIG. 6C**, when the electrode guide driving unit 140 and the pin 152b of the moving unit 152 move to the end of the path provided by the forward movement rail 154, the electrode unit 120 and the electrode guide 130 may be together wound to be close to the tube V in the body as illustrated in **FIG. 3C**. Here, the electrode guide 130 may be in a state where the plurality of joint units 131 is completely drawn out along the curved winding path.

Referring to **FIG. 6D**, when forward movement of the electrode guide driving unit 140 is completed, the pin 152b of the moving unit 152 moves along the connection rail 156 and the hinge 152d rotates. Thus, the connection portion 152a and the tensile force maintaining unit 151 may be disconnected from each other.

After the tensile force maintaining unit 151 is disconnected from the moving unit 152, the electrode connection portion 151d connected to one end of the electrode unit 120 may move in the backward direction. The electrode connection portion 151d may move in the backward direction due to a decrease in tensile force of the first spring 151a. When the electrode connection portion 151d moves in the backward direction, the electrode unit 120 may be brought into contact with the tube V in the body as illustrated in **FIG. 3D****.**

Specifically, as illustrated in **FIG. 6D**, in a state where the protrusion 151b of the tensile force maintaining unit 151 is stopped by the first stopper 151c, only the electrode connection portion 151d of the tensile force maintaining unit 151 moves in the backward direction at the time of disconnection from the moving unit 152. Therefore, the length of the first spring 151a may decrease to a predetermined length (D2→D3), and a tensile force is provided to the electrode unit 120 so that the electrode unit 120 can be brought into close contact with the tube V in the body. That is, when the tensile force maintaining unit 151 and the moving unit 152 are disconnected from each other, only the electrode connection portion 151d of the tensile force maintaining unit 151 moves in the backward direction, and, thus, a distance between one side of the electrode connection portion 151d and one side of the electrode guide driving unit 140 may increase to a predetermined distance (d1→d2).

The electrode unit 120 in contact with the tube V in the body may transfer energy for damaging nerves, and, thus, neurotomy can be performed.

Here, when the tensile force maintaining unit 151 and the moving unit 152 are disconnected from each other, the electrode driving unit 150 may gradually reduce the tensile force of the first spring 151a by means of the reducer 153. That is, the length of the first spring 151a may be gradually decreased to a predetermined length (D2->D3) by means of the reducer 153. Therefore, as the electrode connection portion 151d gradually moves in the backward direction, the electrode unit 120 may be gradually brought into close contact with the tube V in the body. Thus, it is possible to suppress damage to the tube V in the body when the electrode unit 120 is in close contact with the tube V in the body during neurotomy.

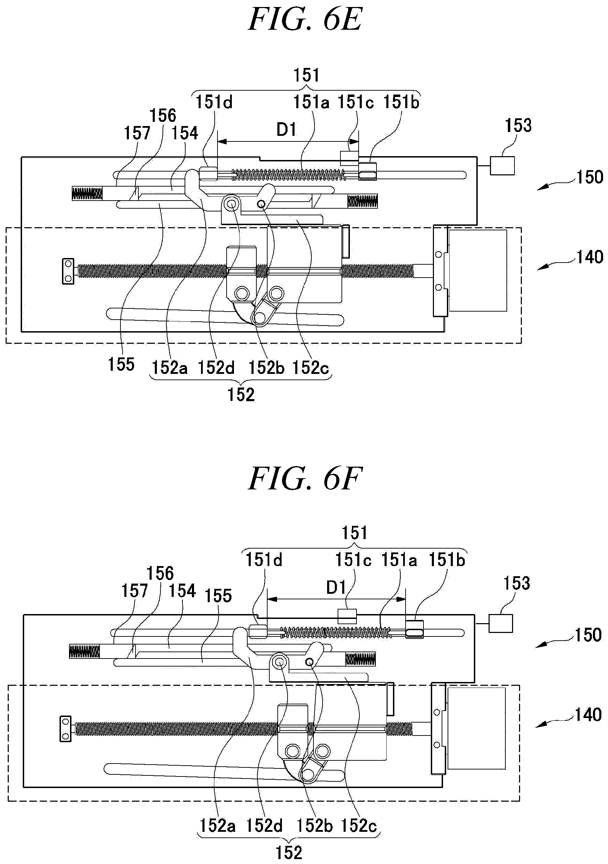

Then, referring to **FIG. 6E** and **FIG. 6F**, after the tensile force maintaining unit 151 and the moving unit 152 are disconnected from each other, the electrode driving unit 150 may move the moving unit 152 in the backward direction. Since the moving unit 152 and the electrode guide driving unit 140 move in the backward direction along the backward movement rail 155, it is possible to make the electrode guide 130 deviate from the circumference of the tube V in the body as illustrated in **FIG. 3E****.**

Specifically, as the electrode guide driving unit 140 moves in the backward direction, the pin 152b of the moving unit 152 connected to the electrode guide driving unit 140 may move in the backward direction along the backward movement rail 155. Thus, the other side of the connection portion 152a meets the electrode connection portion 151d of the tensile force maintaining unit 151 that has moved in the backward direction and thus the other side of the connection portion 152a may cause the tensile force maintaining unit 151 to move in the backward direction.

**As** illustrated in **FIG. 6E**, as the electrode connection portion 151d of the tensile force maintaining unit 151 moves in the backward direction together with the moving unit 152, the length of the first spring 151a may decrease to a predetermined length (D3->D1) and all the tensile force generated in the first spring 151a may be removed.

When the pin 152b moves in the backward direction, the electrode driving unit 150 blocks the connection rail 156 by means of the second stopper 157 to suppress the pin 152b not to move again along the connection rail 156. For example, the second stopper 157 may include a spring that compresses the second stopper 157 in order for the pin 152b to move along the connection rail 156 and returns the second stopper 157 back to its original state when the pin 152b is located on the backward movement rail 155.

**As** the electrode guide driving unit 140 and the electrode driving unit 150 move in the backward direction, the electrode unit 120 and the electrode guide 130 may move in a backward direction B toward the shaft 111 as illustrated in **FIG. 3E**.

When backward movement of the electrode guide driving unit 140 and the electrode driving unit 150 is completed, the pin 152b of the moving unit 152 may be located on the forward movement rail 154, *i.e.,* in a standby state as illustrated in **FIG. 6A****.** Here, the electrode unit 120 and the electrode guide 130 may also be in a standby state before protruding from the shaft 111 as illustrated in **FIG. 3A****.**

Referring to **FIG. 7**, the electrode driving unit 150 according to another embodiment may further include a second spring 158 that connects the support 152c and the connection portion 152a. The electrode driving unit 150 may suppress the pin 152b not to move again along the connection rail 156 by using the second spring 158 when the pin 152b moves in the backward direction.

Therefore, the electrode driving unit 150 may suppress the pin 152b not to move again along the connection rail 156 when the pin 152b moves in the backward direction by using the second spring 158 connecting the support 152c and the connection portion 152a without a stopper that blocks the connection rail 156.

The above description of the present disclosure is provided for the purpose of illustration, and it would be understood by a person with ordinary skill in the art that various changes and modifications may be made without changing technical conception and essential features of the present disclosure. Thus, it is clear that the above-described embodiments are illustrative in all aspects and do not limit the present disclosure. For example, each component described as a single type may be implemented in a dispersed form, and likewise components described as distributed may also be implemented in a combined form.

The scope of the present disclosure is defined by the following claims rather than by the detailed description of the embodiment. It shall be understood that all modifications and embodiments conceived from the meaning and scope of the claims and their equivalents are included in the scope of the present disclosure.

## Claims

1. An electrode device for nerve denervation or modulation in vivo, comprising:
a main body including a shaft;
an electrode unit formed to be drawn out from one end of the shaft and configured to denervate or modulate at least some of nerves on a tube in the body;
an electrode guide coupled to the end of the electrode unit and configured to guide the electrode unit to be brought into contact with the tube in the body;
an electrode guide driving unit configured to move the electrode guide in forward and backward directions; and
an electrode driving unit configured to move the electrode guide in the forward and backward directions in conjunction with the electrode guide driving unit,
wherein the electrode driving unit includes:
a tensile force maintaining unit connected to one end of the electrode unit and configured to provide a tensile force to the electrode unit; and
a moving unit that moves the tensile force maintaining unit in the forward direction until the electrode guide encloses the circumference of the tube in the body in a state where the moving unit is connected to the tensile force maintaining unit, and then is disconnected from the tensile force maintaining unit, and
after the tensile force maintaining unit and the moving unit are disconnected from each other, an electrode connection portion connected to one end of the electrode unit moves in the backward direction.

2. The electrode device of Claim 1,
wherein the tensile force maintaining unit includes:
a first spring that provides a tensile force to the electrode unit, and
the electrode connection portion is moved in the backward direction by the tensile force of the first spring.

3. The electrode device of Claim 2,
wherein the tensile force maintaining unit further includes:
a protrusion protruding from one side; and
a first stopper that blocks movement of the protrusion when the tensile force maintaining unit moves in the forward direction to generate the tensile force of the first spring.

4. The electrode device of Claim 2,
wherein the electrode driving unit further includes:
a reducer that reduces a speed of the electrode connection portion moved in the backward direction by the tensile force of the first spring when the tensile force maintaining unit and the moving unit are disconnected from each other.

5. The electrode device of Claim 1,
wherein when the electrode connection portion is moved in the backward direction, the electrode unit is brought into contact with the tube.

6. The electrode device of Claim 1,
wherein the moving unit further includes:
a connection portion for connection to the tensile force maintaining unit; and
a pin formed in the connection portion and configured to enable the moving unit to move the tensile force maintaining unit in the forward direction, and
the electrode driving unit further includes:
a forward movement rail along which the pin moves in the forward direction.

7. The electrode device of Claim 6,
wherein the electrode driving unit further includes:
a backward movement rail along which the pin moves in the backward direction to make the electrode guide deviate from the circumference of the tube in the body after the moving unit is disconnected from the tensile force maintaining unit.

8. The electrode device of Claim 7,
wherein the forward movement rail and the backward movement rail have the same length.

9. The electrode device of Claim 7,
wherein the moving unit further includes:
a support connected to the electrode guide driving unit; and
a hinge configured to make the connection portion rotate, and
the electrode driving unit further includes:
a connection rail connecting the forward movement rail and the backward movement rail, and
when the pin moves along the connection rail, the hinge rotates and the connection portion is disconnected from the tensile force maintaining unit.

10. The electrode device of Claim 9,
wherein the electrode driving unit further includes:
a second spring that connects the support and the connection portion to suppress the pin not to move again along the connection rail when the pin moves in the backward direction.

11. The electrode device of Claim 9,
wherein the electrode driving unit further includes:
a second stopper that blocks the connection rail when the pin is located on the backward movement rail through the connection rail in order to suppress the pin not to move again along the connection rail when the pin moves in the backward direction.
